# EUROPÄISCHE PATENTANMELDUNG

(11) **EP 0 775 710 A1**
(43) Veröffentlichungstag der Anmeldung: **28.05.1997**
(21) Anmeldenummer: 96118021.3
(22) Anmeldetag: 11.11.1996
(51) Int. Cl.: C07K 1/34

(54) **Verfahren zur Ultrafiltration von Peptide oder Proteine enthaltender biologischer Matrices**

(30) Priorität: 24.11.1995 DE 19543737
(71) Anmelder: HOECHST AKTIENGESELLSCHAFT, 65929 Frankfurt am Main (DE)
(72) Erfinder: Möller, Jörg, Dr., 65812 Bad Soden (DE); Richard, Frank, Dr., 61476 Kronberg (DE)

(57) **Zusammenfassung**

Die vorliegende Erfindung betrifft ein Verfahren zur Vorreinigung mittels Ultrafiltration von komplexen biologischen Matrices, insbesondere von Fermentationsbrühen, welche Peptide oder Proteine enthalten.

## Beschreibung

Die vorliegende Erfindung betrifft ein Verfahren zur Vorreinigung mittels Ultrafiltration von komplexen biologischen Matrices, insbesondere von Fermentationsbrühen, welche Peptide oder Proteine enthalten.

Zur Gewinnung und Reinigung von Proteinen oder Peptiden aus Fermentationsbrühen, beispielsweise mittels chromatographischer Verfahren, muß zunächst eine Vorreinigung durchgeführt werden, die in vielen Fällen aus einer Entsalzung besteht.

Ein Beispiel dafür ist die Gewinnung des Thrombin-Inhibitors-Hirudin, einem einkettigen Polypeptid mit 65 Aminosäuren, aus dem Kulturüberstand eines gentechnisch veränderten Stammes der Hefe Saccharomyces cerevisiae.

Bei dem ursprünglich aus dem Blutegel Hirudo medicinalis isolierten Polypeptid Hirudin handelt es sich um einen hochspezifischen Thrombininhibitor mit breitem therapeutischen Potential (F. Markward, Biomed. Biochim. Acta 44 (1985) 1007 - 1013). Die benötigten Mengen können jedoch nur auf gentechnologischem Wege über transformierte Mikroorganismen hergestellt werden. Dabei hat es sich gezeigt, daß die Hefe Saccharomyces cerevisiae als Wirtsorganismus geeignet ist, um korrekt gefaltetes und voll aktives Hirudin zu produzieren (EP A1 168 342, EP A1 200 655). Die Sezernierung des Proteins ergibt Konzentrationen von bis zu einigen hundert Milligram Hirudin pro Liter Kulturfiltrat. Allerdings ist eine hohe Ausbeute des Proteins nur dann zu erzielen, wenn bei der Hefefermentation komplexe Nährmedien unter Zusatz von Hefeextrakt, Cornsteep, Pepton oder Fleischpaste eingesetzt werden, so daß sich für die Reinigung des Proteins das Problem stellt, Hirudin aus hoher Verdünnung in einem Gemisch von proteinartigen Begleitstoffen zu isolieren.

Zur Entsalzung der so gewonnenen Kulturbrühen und Vorreinigung als Vorbereitung für Chromatographie-Stufen werden neben konventionellen Methoden wie z.B. Extraktion oder Präzipitation auch hydrophobe Adsorptionen/Desorptionen, z.B. an unpolaren Polymermaterialien (auch HIC), verwendet (Atkinson, F. Mavituna; Biochemical Engineering and Biotechnology Handbook, Chapter 16 "Downstream Processing" und Chapter 17 "Product Recovery Processes and Unit Operations", Second Edition, Stockton Press 1991, New York, U.S.A; Brocklebank, M. Kalyanpur: "Primary Separation", Chapter 4 in G. Schmidt-Kastner et al., Hrsg.: "Recovery of Bioproduct", European Federation of Biotechnology, Study Report of Working Party on Downstream Processing, 1993; Müller und W. Brümmer: "Die Chromatographie, eine zentrale Methode in der biotechnischen Aufarbeitung", Chem. Ing.-Tech. 62 (1990) Nr. 5, S. 380-390). Diese Verfahren sind normalerweise dadurch gekennzeichnet, daß teilweise, verglichen mit der Menge herzustellenden Produktes, große Mengen an Lösungsmitteln bzw. an Salzen eingesetzt werden Dies führt zu zusätzlichen Kosten bzw. zu vermehrtem technischen Aufwand für die Wiedergewinnung und/oder Entsorgung der Lösungsmittel oder Salze. Außerdem sind die verbrauchten Adsorptionsharze als Abfall zu beseitigen.

Demgegenüber ist es Aufgabe der vorliegenden Erfindung, zur Vorreinigung, insbesondere zur Entsalzung und Aufkonzentrierung von peptid- oder proteinhaltigen Fermentationsbrühen ein Ultrafiltrationsverfahren mittels Membranen bereitzustellen, welches geeignet ist die Kulturbrühe unter hohem Peptid- bzw. Proteinrückhalt in einem für die nachfolgenden Reinigungsschritte erforderlichen Maß zu entsalzen und aufzukonzentrieren.

Ultrafiltrationsverfahren zur Vorreinigung von Fermentationsbrühen als Vorbereitung für Chromatographiestufen sind auf diesen frühen Verfahrensstufen - außer zum Zweck der Zellabtrennung (T.J. O'Sullivan et al. Chem. Eng. Prog. 80 (1), 68-75 (1984); A. Erikson, Desalination, 53 (1985), 259-263) bislang großtechnisch nicht eingesetzt worden. Ein Grund dafür ist, daß die Membranen von den Nebenprodukten und Begleitstoffen, die vielfältiger Natur sein können, oft blockiert werden und damit zu - für Produktionszwecke - untauglich niedrigen Permeatflüssen führen; damit ergeben sich auch zusätzliche Probleme bei der Reinigung bzw. Regeneration der Membranen (Winzeler: "Membran-Filtration mit hoher Trennleistung und minimalem Energiebedarf", Chimia 44 (1990) 288 - 291). Dies trifft besonders für Herstellverfahren von Proteinen mit niedrigem Molekulargewicht (M < 50,000 Dalton) und Peptiden zu, die vermeintlich Ultrafiltrationsmembranen mit sehr kleinen Trenngrenzen (molecular weight cut-off) erfordern.

Erst auf späteren Verfahrensstufen, wo bereits eine Vorreinigung stattgefunden hat, z.B. zwischen aufeinander folgenden Chromatographie-Stufen, sind Ultrafiltrationen, z.B. zur Entsalzung und Konzentrierung, bereits in der Produktion Stand der Technik.

Ultrafiltrationsverfahren mittels Membranen werden ferner zur Trennung verschieden großer Proteine, zur Pyrogenentfernung oder zur Gewinnung von Biokatalysatoren angewandt (T.J. O'Sullivan et al., Chem. Eng. Prog. 80(1), 68-75 (1984); E. Flaschel et al., Adv. in Biochem. Engineering/Biotechnologie Vol. 26, "Downstream Processing", S. 73-142, N.Y. 1983; Ed.: D.J. Bell).

Den genannten Verfahren liegt sämtlich das Prinzip zugrunde, zur Abtrennung eines Peptids oder eines Proteins eine Membran einzusetzen, deren Trenngrenze (molecular weight cut-off) im oder unter dem Bereich des Molekulargewichts des zurückzuhaltenden Peptids bzw. Proteins liegt.

Es wurde jetzt gefunden, daß es durchaus möglich ist, ein Protein oder Peptide mit einem geringen Molekulargewicht mit sehr hohem Produktrückhalt an Membranen, die eine nominelle Trenngrenze von bis zu 30.000 Dalton (molecular weight cut-off) haben, zu entsalzen und zu konzentrieren.

Demgemäß betrifft die vorliegende Erfindung ein Verfahren zur Vorreinigung einer Peptide oder Proteine enthaltenden, zellfreien Kulturbrühe mittels Ultrafiltration an einer Membran, dadurch gekennzeichnet, daß die angegebene Trenngrenze der Membran den zwei- bis fünffachen, vorzugsweise den drei- bis vierfachen Betrag des Molekulargewichts des von der Membran zurückzuhaltenden Peptids oder Proteins beträgt.

Die vorliegende Erfindung findet insbesondere Anwendung für Vorreinigung von zellfreien Kulturbrühen, die ein rekombinantes Hirudin enthalten, insbesondere solches Hirudin, welches in Saccharomyces cerevisiae zur Expression gebracht wird.

Unter Hirudin sind peptidartige Thrombininhibitoren mit einer spezifischen Aktivität von mindestens 1000 AT-U/mg zu verstehen, die von den bekannten Isohirudinen der Spezies Hirudo medicinalis abgeleitet sind und wesentliche Strukturmerkmale dieser, insbesondere die charakteristische Verknüpfung der drei Disulfidbrücken (J. Dodt et al., Biol. Chem. Hoppe-Seyler 366 (1985) 379-385), aufweisen (vgl. z.B. EP A1 158 564, EP A1 168 342, DE 34 45 517, EP A2 193 175, EP A1 200 655, EP A1 158 986, EP A1 209 061, DE 33 42 199, EP A1 171 024).

Insbesondere wird darunter ein Hirudin verstanden, wie es in der EP A1 171 024, der EP A1 158 986 und der EP A1 209 061 beschrieben ist.

Das erfindungsgemäße Verfahren wird besonders bevorzugt zur Vorreinigung einer zellfreien Kulturbrühe eingesetzt, die ein Hirudinderivat mit der in der EP 0 324 712 offenbarten Aminosäuresequenz enthält ([Leu¹, Thr²]-63-Desulfohirudin).

Bei der Vorreinigung hirudinhaltiger Kulturbrühen beträgt die Trenngrenze der Membran 20 bis 30 kD, vorzugsweise 20 kD.

Vorzugsweise betragen die spezifischen Permeatflüsse bei dem Verfahren gemäß der vorliegenden Erfindung über den gesamten Filtrationsverlauf 10 bis 35 l/m²/h.

Die Temperatur der Kulturbrühe beträgt bei Verwendung von Cornsteep im Fermentationsmedium vorzugsweise 5 bis 15°C.

Bei dem erfindungsgemäßen Verfahren wird vorzugsweise das Permeat solange zur Rohlösung zurückgeführt, bis sich stationäre Verhältnisse, ausgedrückt dadurch, daß die Produktkonzentration im Permeat nicht mehr ansteigt, d.h. konstant ist, eingestellt haben.

### Beispiel

Am Ende der Fermentation eines gentechnisch veränderten Stammes von Saccharomyces cerevisiae in einem komplexen Medium bestehend aus Cornsteep und Hefeextrakt zur Herstellung von Hirudin wird die Kultur zur Inaktivierung der Zellen mit 0,225 ± 0,025 % Benzalkoniumchlorid, z.B. 0,45 ± 0,05 % ® Dodigen 226 (einer 50 %igen Lösung einer Mischung von Alkyldimethyl-benzyl-ammoniumchloriden in Wasser), versetzt und 30 Minuten inkubiert.

Anschließend wird die Brühe zur Abtrennung der Zellen über einen Separator oder Dekanter gefahren und daraufhin über eine 2-stufige Schichtenfiltration in einer Filterpresse, bestehend aus einer Fein- und einer Entkeimungsstufe, geklärt. Das Filtrat wird auf Temperaturen T ≤ 15°C gekühlt, um erneute temperaturbedingte Ausfällungen, die reversibel sind, zu vermeiden.

Das so vorbereitete Filtrat weist eine Leitfähigkeit von χ = 6 ± 0,5 mS/cm auf. Die Ultrafiltrationsanlage bestückt mit Membranen aus Celluloseacetat mit einer Ausschlußgrenze von 20.000 Dalton (z.B. ®Nadir UF-CA-20 in Form von 3,8 Zoll Spiralwickelmodulen) wird zunächst bei folgenden Bedingungen für 60 Minuten unter Permeatrückführung in Betrieb genommen:

| | |
|---|---|
| mittlerer Transmembrandruck | 4 ± 1 bar |
| Volumenstrom pro Druckrohr | 4 - 5 m³/h |

Bei gleichen Bedingungen wird danach unter Permeatableitung 6:1 aufkonzentriert und anschließend durch Zugabe von demineralisiertem und filtriertem Wasser (purified water) unter Konstanthaltung des Volumens solange diafiltriert, d.h. entsalzt, bis die Leitfähigkeit χ ≤ 2,0 mS/cm ist. Im Anschluß an die Diafiltration kann nochmals aufkonzentriert werden, um insgesamt zu einer Konzentrierung von 8:1 zu kommen; dabei soll eine Leitfähigkeit χ < 2,2 mS/cm eingehalten werden.

| Prozeßdaten und Ergebnisse: | |
|---|---|
| Ausgangsvolumen | 4600 l |
| Ausgangskonzentration Hirudin | 100 % |
| Endvolumen | 710 l |
| Endkonzentration Hirudin | 607,2 % |
| Ausgangsleitfähigkeit | 5,83 mS/cm |
| Endleitfähigkeit | 1,97mS/cm |
| Ausbeute an Hirudin | 93,7 % |
| Wiederfindung | 96,9 % |
| Produktverlust im Permeat | 3,3 % |

## Patentansprüche

1. Verfahren zur Vorreinigung einer Peptide oder Proteine enthaltenden, zellfreien Kulturbrühe mittels Ultrafiltration an einer Membran, dadurch gekennzeichnet, daß die angegebene Trenngrenze der Membran den zwei- bis fünffachen Betrag des Molekulargewichts des von der Membran zurückzuhaltenden Peptids oder Proteins beträgt.

2. Verfahren nach Anspruch 1, dadurch gekennzeichnet, daß die angegebene Trenngrenze der Membran den drei- bis vierfachen Betrag des Molekulargewichts des von der Membran zurückzuhaltenden Peptids oder Proteins beträgt.

3. Verfahren nach Anspruch 1 oder 2, dadurch gekennzeichnet, daß das Peptid ein Hirudin ist.

4. Verfahren nach Anspruch 3, dadurch gekennzeichnet, daß die Trenngrenze der Membran 20 bis 30 kD beträgt.

5. Verfahren nach einem der Ansprüche 1 bis 4, dadurch gekennzeichnet, daß die spezifischen Permeatflüsse über den gesamten Filtrationsverlauf 10 bis 35 l/m²/h betragen.

6. Verfahren nach einem der Ansprüche 1 bis 5, dadurch gekennzeichnet, daß bei Verwendung von Cornsteep die Kulturbrühe eine Temperatur von 5 bis 15°C aufweist.

7. Verfahren nach einem der Ansprüche 1 bis 6, dadurch gekennzeichnet, daß das Permeat solange zurückgeführt wird, bis die Produktkonzentration im Permeat einen konstanten Wert angenommen hat.
